# EUROPÄISCHE PATENTANMELDUNG

(11) **EP 2 889 007 A1**
(43) Veröffentlichungstag der Anmeldung: **01.07.2015**
(21) Anmeldenummer: 14200193.2
(22) Anmeldetag: 23.12.2014
(51) Int. Cl.: A61B 17/02

(54) **Sternumoffenhalter**

(30) Priorität: 30.12.2013 DE 202013105963 U
(71) Anmelder: Fehling Instruments GmbH&Co. Kg, 63791 Karlstein (DE)
(72) Erfinder: Fehling, Guido, 63791 Karlstein (DE)
(74) Vertreter: Westphal, Mussgnug & Partner Patentanwälte mbB

(57) **Zusammenfassung**

Die Erfindung betrifft einen Sternumoffenhalter (10, 10', 10", 10"') mit zwei Sternumblättern (20a, 20b), welche gegeneinander verschiebbar angeordnet sind und jeweils eine Anlagefläche (21) aufweisen, wobei der Sternumoffenhalter (10, 10', 10", 10"') ein langgestrecktes längenveränderliches Element (30) aufweist, an dessen Stirnseiten (30a, 30b) die Sternumblätter (20a, 20b) angeordnet sind.

## Beschreibung

Die Erfindung betrifft einen Sternumoffenhalter gemäß dem Oberbegriff des Patentanspruchs 1.

Bekannt sind Sternumoffenhalter mit zwei Sternumblättern, welche gegeneinander verschiebbar angeordnet sind und jeweils eine Anlagefläche aufweisen. Bei den bekannten Sternumoffenhaltern ist dabei in der Regel an jedem der Sternumblätter ein Arm angeordnet, über welchen das Sternumblatt an einer Verschiebemechanik angeordnet ist. Bekannte Sternumoffenhalter sind beispielsweise in der US 5520610 oder 5088472 offenbart.

Der Sternumoffenhalter verbleibt nach erfolgter Operation noch im Körper des Patienten. Die bekannten Sternumoffenhalter werden in der Regel derart angebracht, dass die Sternumblätter durch eine Hautöffnung und einen sich sagittal anschließenden Sternumspalt in den Thorax des Patienten hineinragen, während die Verschiebemechanik auf der Außenseite der Haut angeordnet ist. Dies erschwert ein Verbinden, insbesondere steriles Abdecken, der Wunde.

Die Aufgabe der Erfindung besteht daher darin, einen Sternumoffenhalter bereitzustellen, welcher im in den Patienten eingesetzten Zustand wenig, möglichst gar nicht über die Hautoberfläche nach Außen hervorragt.

Der erfindungsgemäße Sternumoffenhalter mit zwei Sternumblättern, welche gegeneinander verschiebbar angeordnet sind und jeweils eine Anlagefläche aufweisen, zeichnet sich dadurch aus, dass der Sternumoffenhalter ein langgestrecktes längenveränderliches Element aufweist, an dessen Stirnseiten die Sternumblätter angeordnet sind. Das längenveränderliche Element ist erfindungsgemäß nicht mehr abgewinkelt, sondern langgestreckt ausgebildet. Dadurch, dass die Sternumblätter an den Stirnseiten des längenveränderlichen Elements angeordnet sind und somit nicht mehr durch einen Arm quer versetzt zu dem längenveränderlichen Element angeordnet sind, kommt das längenveränderliche Element vollständig zwischen den Sternumblättern zu liegen, wodurch der Sternumoffenhalter möglichst kompakt und flach ausgebildet werden kann. Dadurch wird es insbesondere ermöglicht, dass im in den Patienten eingesetzten Zustand des Sternumoffenhalters das längenveränderliche Element im Bereich zwischen den Sternumblättern angeordnet ist und somit kaum oder nicht über die Hautoberfläche nach Außen hervorsteht. Dies begünstigt ein steriles Abkleben der Wunde.

Gemäß einer besonders bevorzugten Ausführungsform der Erfindung bilden die Anlageflächen zwei gegenüberliegende Seitenflächen eines gedachten Quaders, innerhalb dessen das längenveränderliche Element vollständig angeordnet ist. Dadurch kann der Sternumoffenhalter besonders flach ausgebildet werden und es wird ermöglicht, dass der Sternumoffenhalter nicht über die Hautoberfläche nach Außen hervorragt, sobald er in den Thorax des Patienten eingesetzt ist.

Vorteilhafterweise liegen die Stirnseiten des längenveränderlichen Elements etwa zentral an der Rückseite der Anlageflächen an. Dies ermöglicht eine gute, insbesondere gleichmäßige Kraftübertragung auf die Sternumblätter.

Eine vorteilhafte Ausgestaltung der Erfindung sieht vor, dass die Sternumblätter lösbar an dem längenveränderlichen Element angeordnet sind. Dies ermöglicht eine gute Reinigung des Sternumoffenhalters. Alternativ sind die Sternumblätter unlösbar an dem längenveränderlichen Element angeordnet, was die Zahl der Komponenten verringert.

Gemäß einer bevorzugten Ausführungsform der Erfindung weisen die Sternumblätter an ihrer dem längenveränderlichen Element zugewandten Fläche eine Aufnahme mit unrundem Querschnitt auf, in welche die Stirnseite des längenveränderlichen Elements insbesondere formschlüssig einsetzbar ist. Als Querschnitt ist dabei insbesondere ein Schnitt parallel zur dem längenveränderlichen Element zugewandten Fläche des Sternumblatts durch die Aufnahme anzusehen. Eine unrunde Aufnahme ermöglicht eine Verdrehsicherung. Eine formschlüssige Aufnahme realisiert in einfacher und zuverlässiger Weise eine Fixierung der Sternumblätter an dem längenveränderlichen Element.

Eine besonders bevorzugte Ausführungsform der Erfindung sieht vor, dass die Sternumblätter an ihrer dem längenveränderlichen Element zugewandten Fläche eine zu einer Seite offene Tasche mit einer Deckseite aufweisen, wobei in der Deckseite ein von der offenen Seite ausgehender Schlitz angeordnet ist, und dass an den Stirnseiten des längenveränderlichen Elements eine in die Tasche einschiebbare Eingriffsfläche angeordnet ist. Eine derartige Tasche ermöglicht eine lösbare Verbindung zwischen den Sternumblättern und dem längenveränderlichen Element auf möglichst einfache Art und Weise, nämlich lediglich durch Einschieben. Insbesondere ermöglicht diese Ausgestaltung eine lösbare Verbindung zwischen dem längenveränderlichen Element und den Sternumblättern, ohne den Abstand der Sternumblätter relativ zueinander zum Einsetzen des längenveränderlichen Elements vergrößern zu müssen.

Vorteilhafterweise weist das längenveränderliche Element ein Zentralelement auf, in welchem wenigstens ein Sackloch angeordnet ist, in welchem ein Gewinde angeordnet ist, wobei in das Gewinde des Sacklochs ein Distanzelement mit einem entsprechen Gewinde eingedreht ist. Eine derartige Ausgestaltung des längenveränderlichen Elements mit zwei über ein Gewinde miteinander verbundenen Teilelementen ermöglicht auf einfache Art und Weise eine Längenveränderung, welche kompakt aufgebaut ist.

Gemäß einer besonders bevorzugten Ausführungsform weist das längenveränderliche Element ein Zentralelement auf, in welchem an zwei gegenüberliegenden Stirnseiten jeweils ein Sackloch angeordnet ist, wobei in jedem Sackloch ein Gewinde angeordnet ist, wobei die beiden Gewinde gegenläufig ausgebildet sind und wobei in jedem Sackloch in das Gewinde ein Distanzelement mit einem entsprechenden Gewinde eingedreht ist. Eine derartige Ausgestaltung des längenveränderlichen Elements ermöglicht auf kompakte Art und Weise eine Längenveränderung des längenveränderlichen Elements lediglich dadurch, dass das Zentralelement gegenüber den beiden Distanzelementen verdreht wird. Durch Verwendung zweier Distanzelemente ist insbesondere eine symmetrische Ausgestaltung und eine symmetrische Bewegung der beiden Sternumblätter aufeinander zu oder voneinander weg möglich.

In einer vorteilhaften Ausgestaltung sind die Distanzelemente unlösbar an den entsprechenden Sternumblättern angeordnet, um die Zahl der Komponenten gering zu halten.

Eine bevorzugte Ausführungsform der Erfindung sieht vor, dass das Zentralelement in einem Abschnitt einen unrunden Querschnitt, insbesondere in Form eines Sechskants, aufweist. Dies ermöglicht eine Längenveränderung mittels eines Werkzeugs, beispielsweise eines Schraubenschlüssels, in situ. Weiterhin ermöglicht eine derartige Ausgestaltung eine flache Bauweise des Sternumoffenhalters, bei welchem keine Komponenten im in den Thorax eingesetzten Zustand über die Oberseite der Haut nach Außen vorstehen, da Betätigungselemente des Verschiebemechanismus vermieden werden können.

Vorzugsweise sind die Sternumblätter parallel zueinander angeordnet.

Besonders bevorzugt weisen die Sternumblätter an zwei gegenüberliegenden Längskanten etwa senkrecht zur Anlagefläche jeweils einen Schenkel auf, welcher insbesondere von dem längenveränderlichen Element wegweist. Diese Schenkel vermeiden ein Abrutschen der Sternumblätter an dem Sternum.

Die Erfindung wird anhand der folgenden Figuren ausführlich erläutert. Es zeigen:
- Fig. 1a: eine Ansicht von unten auf ein erstes Ausführungsbeispiel eines erfindungsgemäßen Sternumoffenhalters,
- Fig. 1b: eine stirnseitige Ansicht des Sternumoffenhalters gemäß Fig. 1a,
- Fig. 1c: eine Seitenansicht des Sternumoffenhalters gemäß Fig. 1a,
- Fig. 1d: eine perspektivische Ansicht des Sternumoffenhalters gemäß Fig. 1a,
- Fig. 1e: eine Draufsicht auf den Sternumoffenhalter gemäß Fig. 1a,
- Fig. 1f: einen Schnitt entlang der Linie A-A in Fig. 1e,
- Fig. 1g: einen Schnitt entlang der Linie B-B in Fig. 1e,
- Fig. 2a: eine Ansicht von unten auf ein zweites Ausführungsbeispiel eines erfindungsgemäßen Sternumoffenhalters,
- Fig. 2b: eine stirnseitige Ansicht des Sternumoffenhalters gemäß Fig. 2a,
- Fig. 2c: eine Seitenansicht des Sternumoffenhalters gemäß Fig. 2a,
- Fig. 2d: eine perspektivische Ansicht des Sternumoffenhalters gemäß Fig. 2a,
- Fig. 2e: eine Draufsicht auf den Sternumoffenhalter gemäß Fig. 2a,
- Fig. 2f: einen Schnitt entlang der Linie A-A in Fig. 2e,
- Fig. 2g: einen Schnitt entlang der Linie B-B in Fig. 2e,
- Fig. 3a: eine Ansicht von unten auf ein drittes Ausführungsbeispiel eines erfindungsgemäßen Sternumoffenhalters,
- Fig. 3b: eine stirnseitige Ansicht des Sternumoffenhalters gemäß Fig. 3a,
- Fig. 3c: eine Seitenansicht des Sternumoffenhalters gemäß Fig. 3a,
- Fig. 3d: eine perspektivische Ansicht des Sternumoffenhalters gemäß Fig. 3a,
- Fig. 3e: eine Draufsicht auf den Sternumoffenhalter gemäß Fig. 3a,
- Fig. 3f: einen Schnitt entlang der Linie A-A in Fig. 3e,
- Fig. 3g: einen Schnitt entlang der Linie B-B in Fig. 3e,
- Fig. 4a: eine Ansicht von unten auf ein viertes Ausführungsbeispiel eines erfindungsgemäßen Sternumoffenhalters,
- Fig. 4b: eine stirnseitige Ansicht des Sternumoffenhalters gemäß Fig. 4a,
- Fig. 4c: eine Seitenansicht des Sternumoffenhalters gemäß Fig. 4a,
- Fig. 4d: eine perspektivische Ansicht des Sternumoffenhalters gemäß Fig. 4a,
- Fig. 4e: eine Draufsicht auf den Sternumoffenhalter gemäß Fig. 4a,
- Fig. 4f: einen Schnitt entlang der Linie A-A in Fig. 4e,
- Fig. 4g: einen Schnitt entlang der Linie B-B in Fig. 4e,
- Fig. 5a: eine Draufsicht auf ein fünftes Ausführungsbeispiel eines erfindungsgemäßen Sternumoffenhalters,
- Fig. 5b: eine perspektivische Ansicht des Sternumoffenhalters gemäß Fig. 5a und
- Fig. 5c: einen Schnitt entlang der Linie A-A in Fig. 5a.

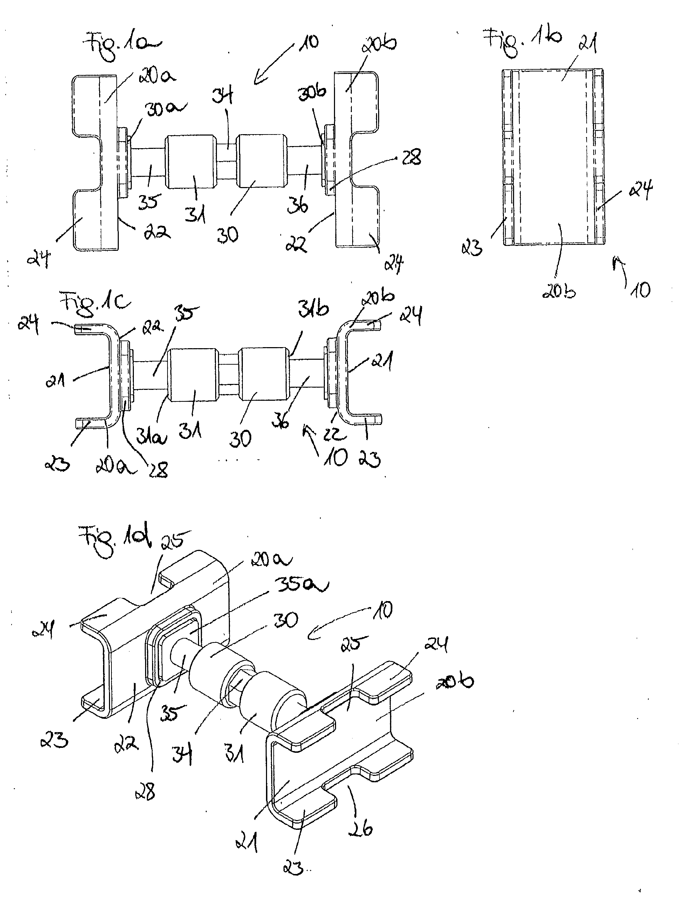

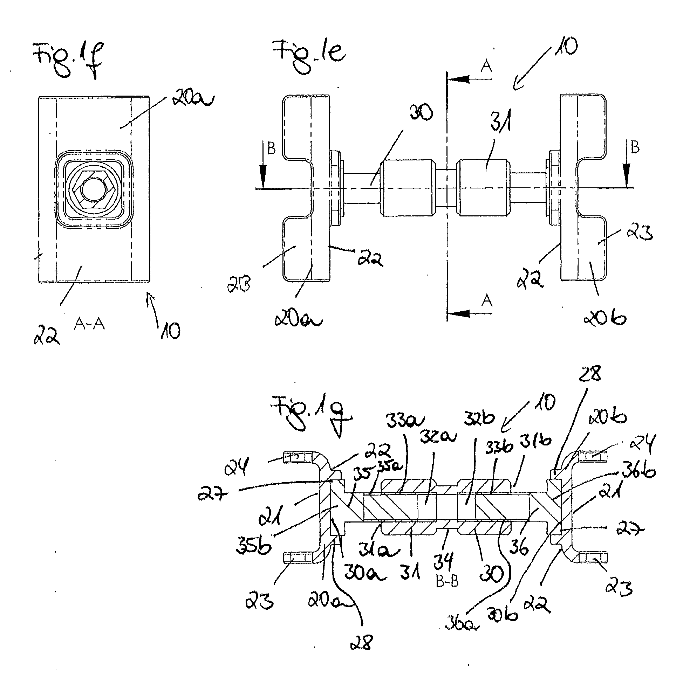

Die Figuren 1a bis 1g zeigen verschiedene Ansichten eines ersten Ausführungsbeispiels eines Sternumoffenhalters 10. Der Sternumoffenhalter 10 weist ein längenveränderliches Element 30 auf, welches langgestreckt mit zwei gegenüberliegenden Stirnseiten 30a, 30b ausgebildet ist. Weiterhin weist der Sternumoffenhalter 10 zwei Sternumblätter 20a, 20b auf, welche an den Stirnseiten 30a, 30b des längenveränderlichen Elements 30 angeordnet sind.

Jedes der Sternumblätter 20a, 20b weist eine Anlagefläche 21 und eine der Anlagefläche 21 gegenüberliegende Rückseite 22 auf.

Die Anlagefläche 21 ist im Wesentlichen rechteckig ausgebildet. Insbesondere sind die Anlageflächen 21 der Sternumblätter 20a, 20b parallel zueinander ausgerichtet. Die Anlageflächen 21 bilden insbesondere zwei gegenüberliegende Flächen eines gedachten Quaders, innerhalb dessen das längenveränderliche Element 30 angeordnet ist. Insbesondere verläuft eine Längsachse des langgestreckten längenveränderlichen Elements 30 im wesentlichen senkrecht zu den Anlageflächen 21 der Sternumblätter 20a, 20b.

An der Anlagefläche 21 der Sternumblätter 20a, 20b können entlang zweier gegenüberliegender Längsseiten, insbesondere an den beiden längeren Längsseiten, jeweils ein Schenkel 23, 24 angeordnet sein, der im Wesentlichen senkrecht zur Anlagefläche 21 ausgerichtet ist und im montierten Zustand von dem längenveränderlichen Element 30 wegweist. Die Schenkel 23, 24 verbessern insbesondere die Anlage des Sternumblatts 20a, 20b an dem Sternum. In einer Ausführungsform können in den Schenkeln 23, 24 insbesondere etwa mittig Ausnehmungen 25, 26 angeordnet sein.

An der Rückseite 22 der Sternumblätter 20a, 20b ist eine Vorrichtung zur vorteilhafterweise lösbaren Befestigung des jeweiligen Sternumblatts 20a, 20b an dem längenveränderlichen Element 30 angeordnet. Diese ist insbesondere etwa zentral oder mittig an der Rückseite 22 der Sternumblätter 20a, 20b angeordnet.

In der in den Figuren 1a bis 1g dargestellten ersten Ausführungsform weist die Befestigungsvorrichtung eine Aufnahme 27 an der Rückseite 22 der Sternumblätter 20a, 20b auf. Die Aufnahme 27 wird insbesondere durch einen auf der Rückseite 22 angeordneten Steg 28 gebildet, welcher die Aufnahme 27 zumindest abschnittsweise, vorteilhafterweise vollständig umschließt. In diesem Ausführungsbeispiel weist die Aufnahme 27 einen im Wesentlichen rechteckigen, insbesondere quadratischen Querschnitt auf, wobei der Querschnitt parallel zur Rückseite 22 liegt. Die Stirnseiten 30a, 30b des längenveränderlichen Elements 30 können in die Aufnahme 27 eingesetzt werden und werden dort klemmend, rastend oder formschlüssig gehalten.

Die Aufnehmung 27 weist einen unrunden Querschnitt auf, um eine Verdrehsicherung zwischen dem Sternumblatt 20a, 20b und dem längenveränderlichen Element 30 bei Befestigung der Sternumblätter 20a, 20b an dem längenveränderlichen Element 30 zu ermöglichen. Als Querschnitt wird dabei insbesondere ein Schnitt parallel zur Rückfläche 22 des entsprechenden Sternumblatts 20a, 20b angesehen.

Das längenveränderliche Element 30 weist ein Zentralelement 31 auf, welches im Wesentlichen als abgesehen von einem unrunden Abschnitt 34 als zylindrisches, insbesondere kreiszylindrisches Element ausgebildet ist. Das Zentralelement 31 weist zwei gegenüberliegende Stirnseiten 31a, 31b auf. In wenigstens einer der Stirnseiten 31a, 31b, insbesondere in beiden der Stirnseiten 31a, 31b, ist ein Sackloch 32a, 32b angeordnet. In dem Sackloch 23a, 23b ist ein Gewinde 33a, 33b angeordnet. Sofern zwei Sacklöcher 32a, 32b mit Gewinden 33a, 33b an dem Zentralelement 31 vorhanden sind, sind vorteilhafterweise die Gewinde 33a, 33b gegenläufig ausgebildet.

In das Sackloch 32a, 32b ist ein Distanzelement 35, 36 eingesetzt. Dazu weist insbesondere das Distanzelement 35, 36 einen Abschnitt mit einem Gewinde 35a, 36a auf, welches in das Gewinde 33a, 33b des entsprechenden Sacklochs 32a, 32b eingedreht werden kann. An dem Distanzelement 35, 36 kann ein Kopf 35b, 36b ausgebildet sein, welcher zur Befestigung an dem Sternumblatt 20a, 20b ausgebildet ist. Die Köpfe 35b, 36b der Distanzelemente 35, 36 bilden insbesondere die Stirnflächen 30a, 30b des längenveränderlichen Elements 30.

In der vorliegenden Ausführungsform ist der Kopf 35b, 36b insbesondere derart ausgebildet, dass er klemmend oder formschlüssig in der Aufnahme 27 aufgenommen werden kann. Dazu ist beispielsweise der Kopf 35b, 36b als flaches, im Wesentlichen quadratisches Element ausgebildet.

Der unrunde Abschnitt 34 des Zentralelements 31 weist insbesondere die Außenkontur eines Sechskants auf, so dass ein Schraubenschlüssel an dem Zentralelement 31 angesetzt werden kann. Beim Drehen des Zentralelements 31 beispielsweise mittels des Schraubenschlüssels wird das Zentralelement 31 gegen die Distanzelemente 35, 36 verdreht. Da die Distanzelemente 35, 36 winkelstabil an den Sternumblättern 20a, 20b in den Aufnahmen 27 gehalten sind, werden durch Drehen des Zentralelements 31 die Distanzelemente 35, 36 je nach Drehrichtung entweder beide gleichzeitig aus den entsprechenden Sacklöchern 32a, 32b heraus oder in die Sacklöcher 32a, 32b hineingedreht, wodurch eine Längenvariation des längenveränderlichen Elements 30 erreicht wird.

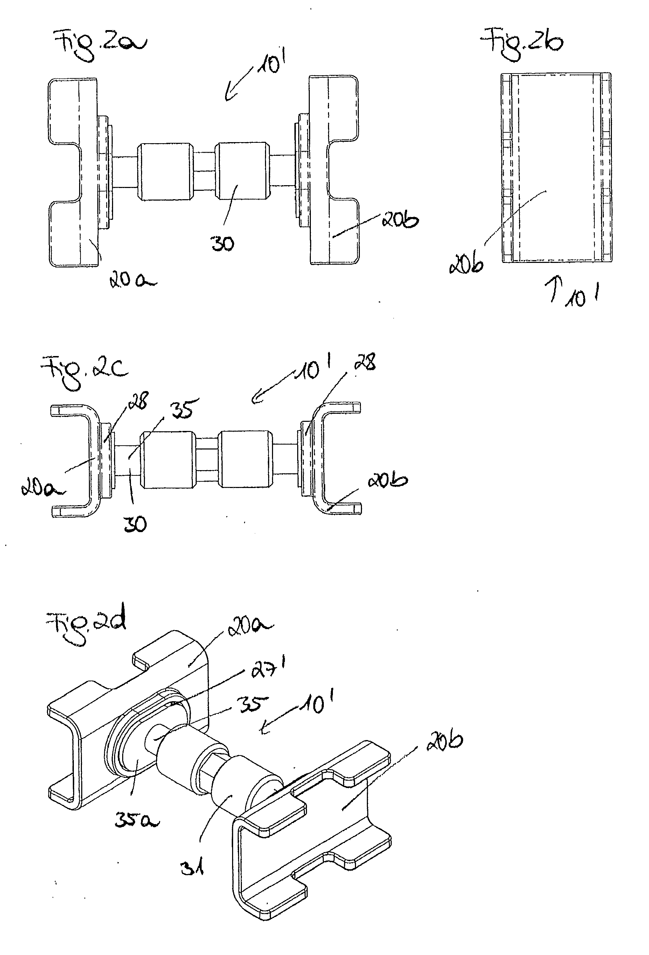

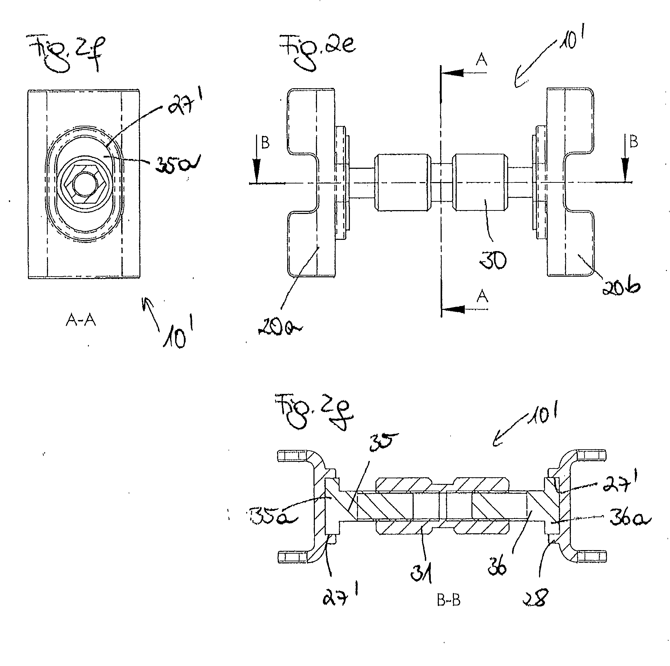

Das in den Figuren 2a bis 2g dargestellte weitere Ausführungsbeispiel eines Sternumoffenhalters 10' unterscheidet sich von dem in den Figuren 1a bis 1g dargestellten Sternumoffenhalter 10 lediglich in der Ausgestaltung der Aufnahme 27'. Diese weist in dem zweiten Ausführungsbeispiel des Sternumoffenhalters 10' einen Querschnitt in Form eines Ovals, einer Ellipse, eines Rechtecks mit abgerundeten Ecken oder eines Rechtecks mit an zwei gegenüberliegenden Stirnseiten angesetzten Halbkreisen oder Kreiskappen auf. Entsprechend sind die Köpfe 35b, 36b der Distanzelemente 35, 36 mit zu der Form der Ausnehmung 27' korrespondierender Form ausgebildet.

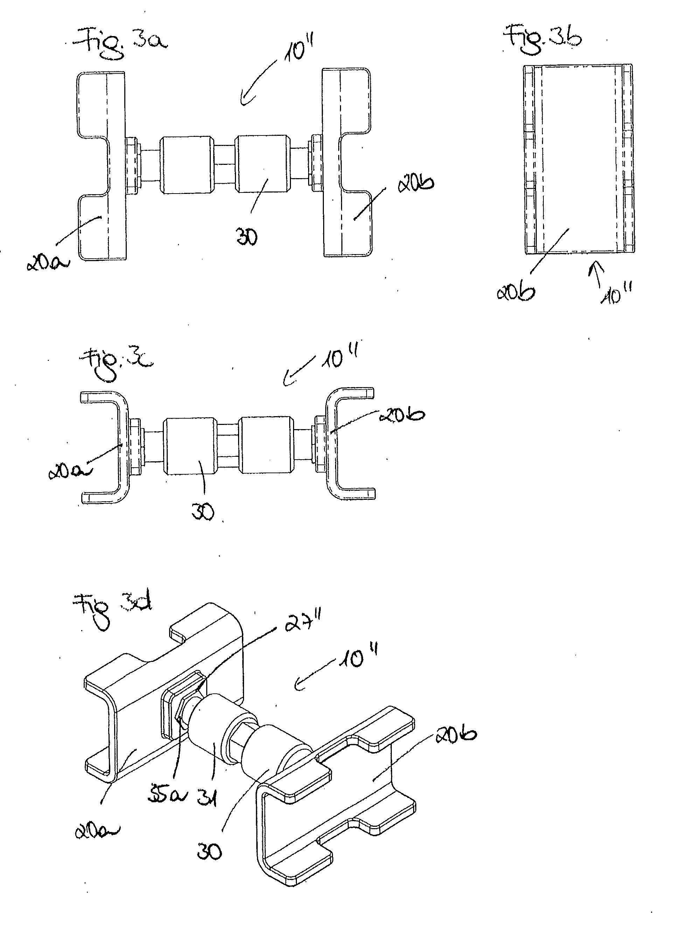

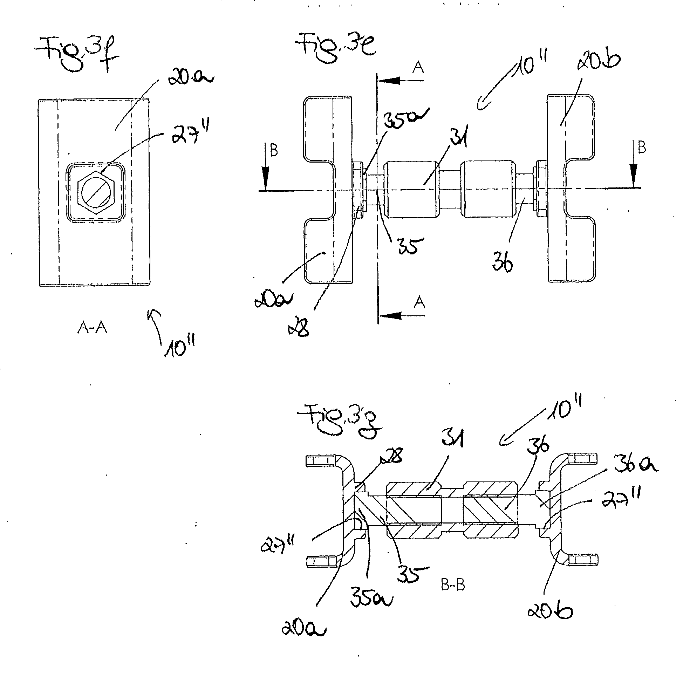

Das in den Figuren 3a bis 3g dargestellte Ausführungsbeispiel eines Sternumoffenhalters 10" unterscheidet sich von dem in den Figuren 1a bis 1g dargestellten Sternumoffenhalter 10 lediglich in der Ausgestaltung der Ausnehmung 27". Die Ausnehmung 27" weist einen sechseckigen Querschnitt auf, in welchen beispielsweise der Kopf 35b, 36b einer Schraube einsetzbar ist, so dass die Distanzelemente 35, 36 durch handelsübliche Schrauben gebildet werden können.

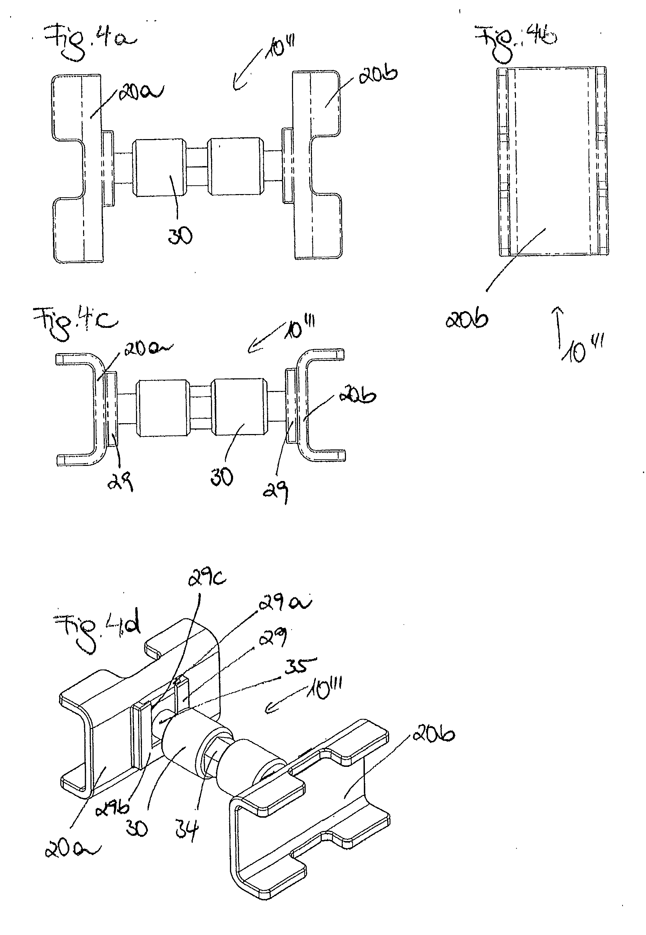

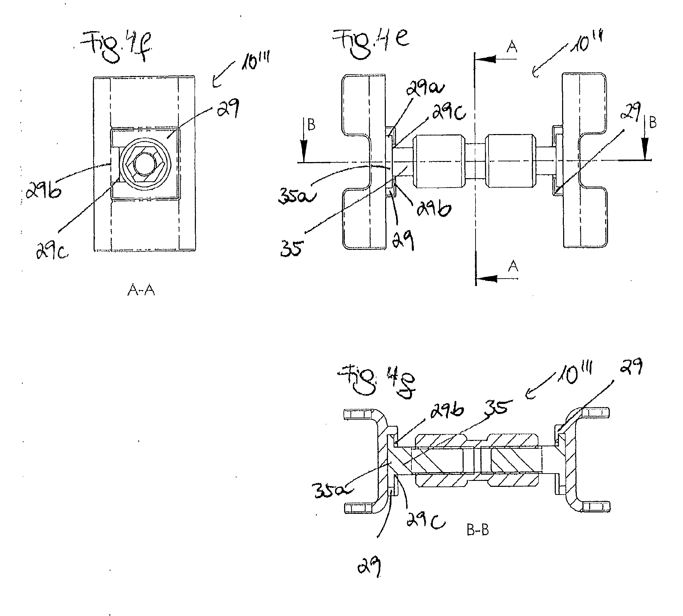

Das in den Figuren 4a bis 4g dargestellte Ausführungsbeispiel eines Sternumoffenhalters 10''' unterscheidet sich von dem in den Figuren 1a bis 1g dargestellten Sternumoffenhalter 10 durch die Ausgestaltung der Befestigung zwischen dem längenveränderlichen Element 30 und den Sternumblättern 20a, 20b. Die Sternumblätter 20a, 20b gemäß dem vierten Ausführungsbeispiel weisen auf ihrer Rückseite 22 eine zu einer Seite 29a offene Tasche 29 auf. Die Tasche 29 weist eine Deckseite 29b auf, welche im Wesentlichen parallel zu der Rückseite 22 angeordnet ist. In der Deckseite 29b ist ausgehend von der offenen Seite 29a ein Schlitz 29c angeordnet. Die Tasche 29c weist parallel zu der Rückseite 22 einen unrunden Querschnitt auf, welcher vorliegend rechteckig oder quadratisch ausgebildet ist. Die Köpfe 35b, 36b der Distanzhalter 35, 36 dieses Ausführungsbeispiels weisen eine zur Form der Tasche korrespondierende Form auf, und sind beispielsweise ebenfalls rechteckig oder quadratisch ausgebildet. Die Kopfe 35b, 36b können von der offenen Seite 29a her in die Tasche 29 eingeschoben werden, und bildet auf diese Weise ein Eingriffselements mit der Tasche 29. Dabei ragt das Distanzelement 35, 36 durch den Schlitz 29c aus der Tasche 29 hervor, insbesondere im Wesentlichen senkrecht zur Rückseite 22 des Sternumblatts 20a, 20b. Durch den unrunden Querschnitt der Taschen 29 und der Köpfe 35b, 36b, die aufeinander abgestimmt sind, kann eine Verdrehsicherung der Sternumblätter 20a, 20b gegenüber der Distanzelemente 35, 36 ermöglicht werden.

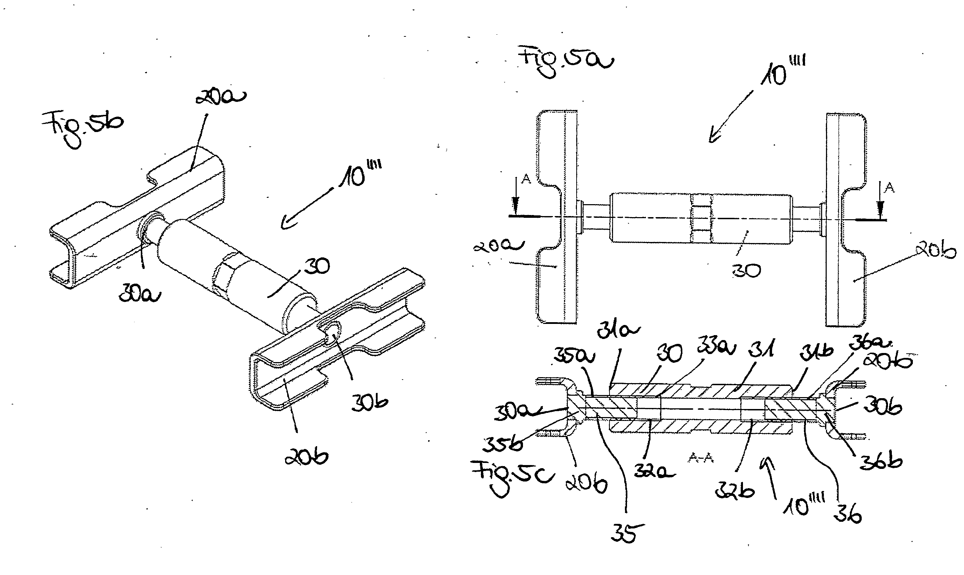

Das in den Figuren 5a bis 5c dargestellte Ausführungsbeispiel eines Sternumoffenhalters 10'''' unterscheidet sich von dem in den Figuren 1a bis 1g dargestellten Sternumoffenhalter 10 durch die Ausgestaltung der Befestigung zwischen dem längenveränderlichen Element 30 und den Sternumblättern 20a, 20b. In diesem Ausführungsbeispiel sind die Stirnseiten 30a, 30b des längenveränderlichen Elements 30 fest, d. h. unlösbar, an den Sternumblättern 20a, 20b fixiert, beispielsweise durch Löten oder Schweißen. Insbesondere ist dazu der Kopf 35b, 36b der Distanzelemente 35, 36 an dem entsprechenden Sternumblatt 20a, 20b fixiert, beispielsweise durch Löten oder Schweißen. Dabei kann der Kopf 35b, 36b sowohl mit seiner Stirnseite flächig an dem entsprechenden Sternumblatt 20a, 20b fixiert sein als auch in eine Ausnehmung des entsprechenden Sternumblatts 20a, 20b eingesetzt und dort fixiert sein, wie in den Figuren 5a bis 5c dargestellt.

### Bezugszeichenliste

- 10: Sternumoffenhalter
- 10': Sternumoffenhalter
- 10'': Sternumoffenhalter
- 10''': Sternumoffenhalter
- 10'''': Sternumoffenhalter

- 20a: Sternumblatt
- 20b: Sternumblatt
- 21: Anlagefläche
- 22: Rückseite
- 23: Schenkel
- 24: Schenkel
- 25: Ausnehmung
- 26: Ausnehmung
- 27: Aufnahme
- 27': Aufnahme
- 27'': Aufnahme
- 28: Steg
- 29: Tasche
- 29a: Seite
- 29b: Deckseite
- 29c: Schlitz

- 30: Längenveränderliches Element
- 30a: Stirnseite
- 30b: Stirnseite
- 31: Zentralelement
- 31a: Stirnseite
- 31b: Stirnseite
- 32a: Sackloch

- 32b: Sackloch
- 33a: Gewinde
- 33b: Gewinde
- 34: Abschnitt
- 35: Distanzelement
- 35a: Gewinde
- 35b: Kopf
- 36: Distanzelement
- 36a: Gewinde
- 36b: Kopf

## Patentansprüche

1. Sternumoffenhalter (10, 10', 10'', 10''') mit zwei Sternumblättern (20a, 20b), welche gegeneinander verschiebbar angeordnet sind und jeweils eine Anlagefläche (21) aufweisen,
**dadurch gekennzeichnet, dass** der Sternumoffenhalter (10, 10', 10'', 10''') ein langgestrecktes längenveränderliches Element (30) aufweist, an dessen Stirnseiten (30a, 30b) die Sternumblätter (20a, 20b) angeordnet sind.

2. Sternumoffenhalter nach Anspruch 1,
**dadurch gekennzeichnet, dass** die Anlageflächen (21) zwei gegenüberliegende Seitenflächen eines gedachten Quaders bilden, innerhalb dessen das längenveränderliche Element (30) vollständig angeordnet ist.

3. Sternumoffenhalter nach Anspruch 1 oder 2,
**dadurch gekennzeichnet, dass** die Stirnseiten (30a, 30b) des längenveränderlichen Elements (30) etwa zentral an der Rückseite (22) der Anlageflächen (21) anliegen.

4. Sternumoffenhalter nach einem der vorhergehenden Ansprüche,
**dadurch gekennzeichnet, dass** die Sternumblätter (20a, 20b) unlösbar oder lösbar an dem längenveränderlichen Element (30) angeordnet sind.

5. Sternumoffenhalter nach einem der vorhergehenden Ansprüche,
**dadurch gekennzeichnet, dass** die Sternumblätter (20a, 20b) an ihrer dem längenveränderlichen Element (30) zugewandten Fläche eine Aufnahme (27, 27', 27") mit unrundem Querschnitt aufweisen, in welche die Stirnseite (30a, 30b) des längenveränderlichen Elements (30) insbesondere formschlüssig einsetzbar ist.

6. Sternumoffenhalter nach einem der vorhergehenden Ansprüche,
**dadurch gekennzeichnet, dass** die Sternumblätter (20a, 20b) an ihrer dem längenveränderlichen Element (30) zugewandten Fläche eine zu einer Seite (29a) offene Tasche (29) mit einer Deckseite (29b) aufweisen, wobei in der Deckseite (29b) ein von der offenen Seite (29a) ausgehender Schlitz (29c) angeordnet ist, und dass an den Stirnseiten (30a, 30b) des längenveränderlichen Elements (30) eine in die Tasche (29) einschiebbare Eingriffsfläche angeordnet ist.

7. Sternumoffenhalter nach einem der vorhergehenden Ansprüche,
**dadurch gekennzeichnet, dass** das längenveränderliche Element (30) ein Zentralelement (31) aufweist, in welchem wenigstens ein Sackloch 32a, 32b) angeordnet ist, in welchem ein Gewinde (33a, 33b) angeordnet ist, wobei in das Gewinde (33a, 33b) des Sacklochs (32a, 32b) ein Distanzelement (35, 36) mit einem entsprechenden Gewinde (35aa, 35b) eingedreht ist.

8. Sternumoffenhalter nach einem der vorhergehenden Ansprüche,
**dadurch gekennzeichnet, dass** das längenveränderliche Element (30) ein Zentralelement (31) aufweist, in welchem an zwei gegenüberliegenden Stirnseiten (31a, 31b) jeweils ein Sackloch (32a, 32b) angeordnet ist, wobei in jedem Sackloch (32a, 32b) ein Gewinde (33a, 33b) angeordnet ist, wobei die beiden Gewinde (33a, 33b) gegenläufig ausgebildet sind und wobei in jedem Sackloch (32a, 32b) in das Gewinde (33a, 33b) ein Distanzelement (35, 36) mit einem entsprechenden Gewinde (35a, 35b) eingedreht ist.

9. Sternumoffenhalter nach einem der Ansprüche 7 bis 8,
**dadurch gekennzeichnet, dass** das Zentralelement (31) in einem Abschnitt (34) einen unrunden Querschnitt, vorzugsweise in Form eines Sechskants, aufweist.

10. Sternumoffenhalter nach einem der Ansprüche 7 bis 9,
**dadurch gekennzeichnet, dass** das Distanzelement (35, 36) unlösbar an dem Sternumblatt (20a, 20b) angeordnet ist.

11. Sternumoffenhalter nach einem der vorhergehenden Ansprüche,
**dadurch gekennzeichnet, dass** die Sternumblätter (20a, 20b) parallel zueinander angeordnet sind.

12. Sternumoffenhalter nach einem der vorhergehenden Ansprüche,
**dadurch gekennzeichnet, dass** die Sternumblätter (20a, 20b) an zwei gegenüberliegenden Längskanten etwa senkrecht zur Anlagefläche (21) jeweils einen Schenkel (23, 24) aufweisen.
